(19) Europäisches Patentamt
European Patent Office
Office européen des brevets

(11) Veröffentlichungsnummer: **0 108 181**
**B1**

(12) # EUROPÄISCHE PATENTSCHRIFT

(45) Veröffentlichungstag der Patentschrift: **29.04.87**

(51) Int. Cl.⁴: **C 07 D 257/10** // C07F15/00

(21) Anmeldenummer: **82810468.7**

(22) Anmeldetag: **03.11.82**

(54) **Dibenzo(b,i)-1,4,8,11-tetraaza(14)annulenkobalt(III)jodid.**

(43) Veröffentlichungstag der Anmeldung: **16.05.84 Patentblatt 84/20**

(45) Bekanntmachung des Hinweises auf die Patenterteilung: **29.04.87 Patentblatt 87/18**

(84) Benannte Vertragsstaaten: **DE**

(56) Entgegenhaltungen:
**DE-A-1 569 667**
**DE-A-2 214 336**
**FR-A-1 576 624**

**JUSTUS LIEBIGS: "Annalen der Chemie", Band 717, 1968, Verlag Chemie GmbH, Weinheim, DE. H. HILLER et al.: "5.14-Dihydro-dibenzo b.i. 5.9.14.18 tetraazal 14 annulen, ein makrocyclischer Chelat-Bildner"**
**Journal of the American Chemical Society, Vol. 99, No. 1, 1977, Seiten 286-288**

(73) Patentinhaber: **CIBA- GEIGY AG, Klybeckstrasse 141, CH- 4002 Basel (CH)**

(72) Erfinder: **Hunziker, Max, Dr., St. Alban Ring 230, CH- 4052 Basel (CH)**
Erfinder: **Hilti, Bruno, Dr., Marschalkenstrasse 27, CH- 4054 Basel (CH)**

**EP 0 108 181 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Dibenzo[b,i]-1,4,8,11-tetraaza[14]annulenkobalt(III) ist bekannt (H. Hiller et al., Liebigs Ann. Chem. 717 (1968)). Ferner ist es bekannt, z.B. Kobaltphthalocyanin mit Joddampf zu oxidieren (B.M. Hoffmann et al., J. Am. Chem. Soc. 99, 286 (1977)).

Gegenstand der Erfindung ist der neue Komplex der Formel 1

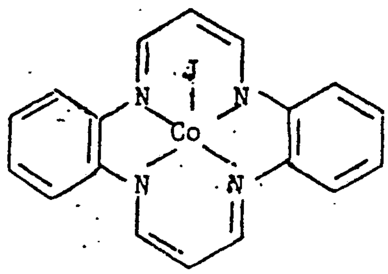

(I) ,

d.h. das Dibenzo[b,i]-1,4,8,11-tetraaza[14]annulenkobalt(III)jodid. In diesem Komplex weist Co(III) die seltene Koordinationszahl 5 (quadratisch-pyramidal) auf. Die Packung der Moleküle im Kristall ist derart, dass keine von Co(III) üblicherweise angenommene oktaedrische oder pseudooktaedrische Koordinationssphäre entsteht. Die quadratischpyramidale Koordination des Co(III) bleibt streng erhalten.

Der erfindungsgemässe Komplex kann in Form eines mikrokristallinen Pulvers und insbesondere in Form von Einkristallen vorliegen. Der Komplex der Formel I kann durch Umsetzung in der Gasphase (Sublimation) hergestellt werden. Dabei lässt man das Dibenzo[b,i]-1,4,8,11-tetraaza[14]annulenkobalt(II) [hergestellt gemäss H. Hiller et al., Liebigs Ann. Chem., 717, 137 (1968)] und das Jod zweckmässig in einer Inertgasatmosphäre bei erhöhter Temperatur miteinander reagieren, bevorzugt in offenem System. Die Umsetzung in der Gasphase kann aber auch in geschlossenem System in Inertgasatmosphäre vorgenommen werden. Dieses Verfahren ermöglicht die Herstellung von sehr reinen Kristallen, die praktisch keine Verunreinigungen, wie z.B. Lösungsmittelmoleküle, enthalten. Die durch Sublimation erhaltenen Kristalle zeichnen sich ferner durch optisch einwandfreie Oberflächen aus.

Figur 1 veranschaulicht eine für die Umsetzung in der Gasphase besonders geeignete Versuchsanordnung. Dabei werden das Dibenzo[b,i]-1,4,8,11-tetraaza[14]annulenkobalt(II) [DBTAACo] und das Jod in getrennten, mit Heizelementen (3) bzw. mit einem Thermostaten (4) ausgerüsteten, evakuierten Kammern (1, 2) verdampft und dann durch die Trägergase TG 1 (für das Dibenzo[b,i]-1,4,8,11-tetraaza[14]annulen-kobalt(II)) und TG 2 (für das Jod) in die Reaktionskammer (5) eingeführt. Die Kristalle des erfindungsgemässen Komplexes wachsen in der Reaktionskammer (6) entweder an den Reaktorwänden oder auf einem in der Reaktionskammer angeordneten Substrat. Die Reaktionsgefässe können z.B. aus Pyrexglas, bevorzugt jedoch aus Quarz, gefertigt sein.

Als Trägergase verwendet man zweckmässig hochreine Inertgase, wie Argon, Helium oder Xenon. TG 1 und TG 2 in der Versuchsanordnung gemäss Abbildung 1 können verschieden oder gleich gewählt werden. Bevorzugt wird sowohl für das Dibenzo[b,i]-1,4,8,11-tetraaza[14]annu-lenkobalt(II) als auch für das Jod hochreines Argon eingesetzt.

Als Substrate, auf denen sich die Kristalle bilden, kann beispielsweise Aluminiumoxid oder bevorzugt Quarz in beliebiger Form, z.B. in Form von Stäben, Rohren und dergl., eingesetzt werden.

Vorzugsweise wird die Umsetzung in der Gasphase bei einer Temperatur zwischen 200 und 300°C und einem Totaldruck zwischen 100 Pa und 3 kPa vorgenommen. Die Reaktionskomponenten werden in mindestens stöchiometrischer Menge eingesetzt. Bevorzugt verwendet man aber einen Überschuss an Jod, zweckmässigerweise einen etwa 10- bis 100-fachen molaren Überschuss. Die durch Sublimation erhaltenen Kristalle lassen sich leicht aus der Reaktionszone bzw. vom Substrat entfernen.

**Beispiel:**

Der Versuch wird in einer Anordnung gemäss Figur 1 durchgeführt. In der Kammer 1 werden 0,1 g ($2{,}9 \times 10^{-4}$ Mol) Dibenzo[b,i]-1,4,8,11-tetraaza[14]annulenkobalt(II) und in der Kammer 2 5 g (0,04 Mol) Jod ("Suprapur" der Fa. Merck, Darmstadt, BRD) vorgelegt. Durch Anlegen eines Vakuums und einer regelbaren Inertgaszufuhr [Argon 99,9997% vpM (volume per million)] wird ein Totaldruck von 950 Pa eingestellt. Dann werden die beiden Reaktanden durch die getrennt regelbaren Heizelemente (3) und (4) verdampft [Verdampfungstemperatur des Dibenzo[b,i]-1,4,8,11-tetraaza[14]annulenkobalt(II) = 220-260°C; Verdampfungstemperatur des Jods 20-50°C],

in der Kammer (5) zur Reaktion gebracht und durch das Inertgas zur unbeheizten Zone (6) transportiert, wo Einkristalle des erfindungsgemässen Komplexes wachsen.

Die Wachstumszeit für Einkristalle von ca. 1 $mm^3$ liegt je nach eingestellter Verdampfungstemperatur zwischen 2 und 10 Tagen. Die anfallenden schwarzen, im Auflicht stahlgrauen Kristalle können direkt für die Röntgenstrukturanalyse verwendet werden. Der Komplex der Formel I ist nicht-leitend.

Elementaranalyse für $C_{18}H_{14}CoJN_4$:
berechnet C 45,79 H 2,99 N 11,87 J 26,88
gefunden C 45,72 H 3,00 N 11,92 J 27,04.

Dichte: 1,973 g/$cm^3$ experimentell, 1,971 g/$cm^3$ aus Röntgendaten berechnet. Kristalldaten (an Einkristallen mittels Diffraktometrie gemessen) $MoK_{\alpha 1}$ = 0,70926 Å, F.W. 472,2: monoklin, Raumgruppe $P2_1/c$, a = 10,001(4)Å, b = 12,916(5) Å, c = 13,848(5) Å, β = 117,18°, Z = 4. Die Kristallstruktur wird mit direkten Methoden unter Verwendung von 2449 Reflexen mit I > 2δ(I) gelöst und kann mit anisotropen Temperaturparametern für alle Nicht-Wasserstoffatome bis zu einem R-Wert von 0,032 verfeinert werden.

**Patentansprüche**

1. Der Komplex der Formel I

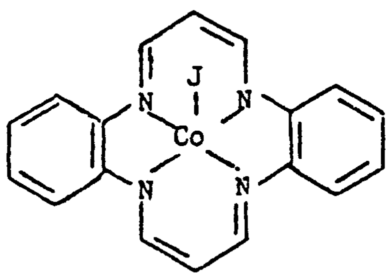

(I) .

2. Dibenzo[b,i]-1,4,8,11-tetraaza[14]annulenkobalt(III)jodid nach Anspruch 1 in Einkristallform.

3. Verfahren zur Herstellung des Komplexes der Formel 1 nach Anspruch 1, dadurch gekennzeichnet, dass man Dibenzo[b,i]-1,4,8,11-tetraaza[14]annulenkobalt(II) und Jod in der Gasphase in einer Inertgasatmosphäre bei erhöhter Temperatur miteinander reagieren lässt.

4. Verfahren nach Anspruch 3, dadurch gekennzeichnet, dass man die Umsetzung in der Gasphase bei einer Temperatur zwischen 200 und 300°C und einem Totaldruck zwischen 100 Pa und 3 kPa vornimmt.

**Claims**

1. The complex of formula I

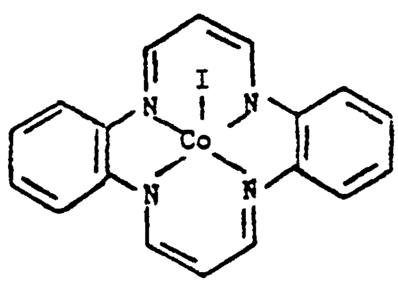

(I) .

2. Dibenzo[b,i]-1,4,8,11-tetraaza[14]annulenecobalt(III) iodide according to claim 1 in the form of a monocrystal.

3. A process for the preparation of the complex of formula I according to claim 1, which process comprises reacting with each other dibenzo[b,i]-1,4,8,11-tetraaza[14]annulenecobalt(II) and iodine in the gas phase in an inert gas atmosphere at elevated temperature.

4. A process according to claim 3, which comprises performing the reaction in the gas phase at a temperature in the range from 200°C to 300°C and under a total pressure in the range from 100 Pa to 3 kPa.

**Revendications**

1. Le complexe de formule I

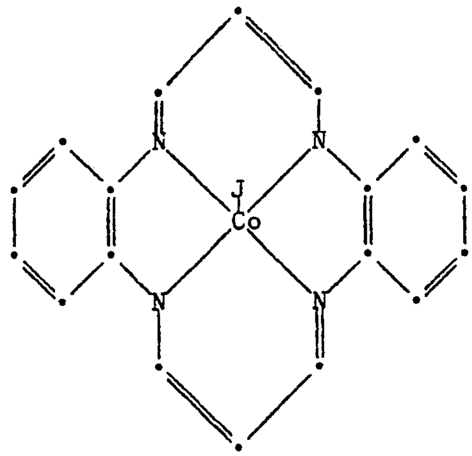

(I).

2. Iodure de cobaltico (III) dibenzo [b,i]-1,4,8,11-tétraaza[14]-annulène.

3. Procédé de préparation du complexe de formule I selon la revendication 1, caractérisé en ce qu'on laisse réagir entre eux de l'iode et du dibenzo[b,i]-1,4,8,11-tétraaza[14]annulène-cobalt (II) en phase gazeuse dans une atmosphère de gaz inerte à haute température.

4. Procédé selon la revendication 3, caractérisé en ce que la réaction se fait en phase gazeuse à une température entre 200 et 300°C et à une pression totale entre 100 Pa et 3 kPa.

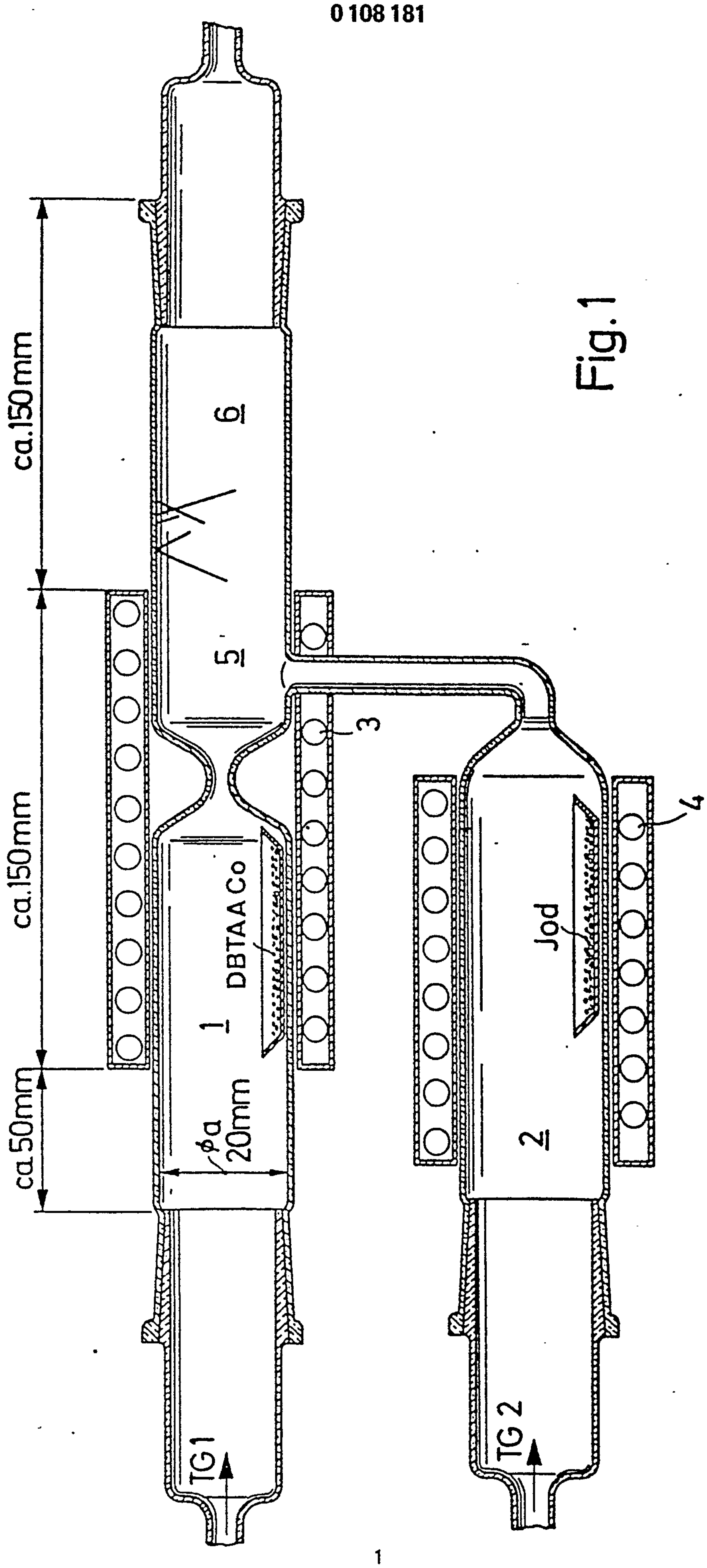


Fig. 1